# EUROPEAN PATENT APPLICATION

(11) **EP 0 739 978 A2**
(43) Date of publication of application: **30.10.1996**
(21) Application number: 96250090.6
(22) Date of filing: 24.04.1996
(51) Int. Cl.: C12N 9/86, C12N 1/20, C12P 13/02, C12P 41/00, C12P 13/04

(54) **Hydantoinase from agrobacterium tumefaciens and its use for the preparation of optically pure N-carbamyl-D-amino acids from racemic hydantoins**

(30) Priority: 28.04.1995 US 431352
(71) Applicant: W.R. Grace & Co.-Conn., New York New York 10036 (US)
(72) Inventor: Weber, Jennifer Elizabeth, Woodstock, MD 21163 (US); Durham, Donald Richard, Gaithersburg, Maryland 20760 (US)
(74) Representative: UEXKÜLL & STOLBERG

(57) **Abstract**

A novel hydantoinase exhibiting high activity and stability in alkaline medium at high temperatures and produced from *Agrobacterium tumefaciens* ATCC 55632 is disclosed. The hydantoinase is well suited for the bioconversion of racemic DL-hydantoins to optically pure N-carbamyl D-amino acids. The process for production of optically pure compounds is also disclosed.

## Description

### Field of the Invention

The field of the present invention relates to a hydantoinase produced by a newly isolated strain of *Agrobacterium tumefaciens* and characterized by substantial activity and stability in alkaline medium and at high temperatures. The hydantoinase is useful for the stereospecific conversion of racemic DL-hydantoin compounds to N-carbamyl D-amino acids.

### Background of the Invention

Hydantoinase is an enzyme that catalyzes the conversion of 5-monosubstituted hydantoins to N-carbamyl amino acids. The N-carbamyl amino acid can be converted to an optically pure preparation of D-amino acid either by chemical methods or by a second enzymatic step catalyzed by an N-carbamyl-amino acid amino hydrolase. It is well known in the art (Syldatk, et al., Biocatalytic Production of Amino Acids and Derivatives, D. Rozzell and F. Wagner, eds. pp. 77-128, 1992) that optically pure D-amino acids and their derivatives are useful as chiral substrates for the chemical synthesis of semisynthetic penicillins, cephalosporins, biologically active peptides, pesticides, synthetic sweeteners and pharmaceutical products such as analogs for excitatory amino acid antagonists (J. Org. Chem. 58:7263, 1993).

Hydantoinases have been isolated from a broad category of organisms. Wallach and Grisolia report that hydantoinases have been isolated from calf, rat, and pigeon liver (J. Biol. Chem. 226:277-288, 1957). Esaki, et al. report that hydantoinases have been found in a variety of microorganisms, particularly in *Klebsiella*, *Corynebacterium*, *Agrobacterium*, *Pseudomonas*, *Bacillus*, and *Streptomyces*. (Biotechnology and Bioengineering XXII, Supplement 1, 127-141, 1980). Nakamori, et al. also describe a variety of microbial hydantoinases in U.S. Patent 4,211,840.

Hydantoinase has been isolated from *Agrobacterium* species. Yamada, et al. describe the preparation of a hydantoinase from a variety of bacterial strains (see Table 1 of U.S. patents 4,094,741 and 4,237,227) including *Agrobacterium*. Makryaleas, et al. describe a hydantoinase isolated from *Agrobacterium radiobacter* in U.S. patent 4,980,284. Yamada, et al. (Fermentation Technology 56[5]:404-491, 1978) also describe hydantoinases from a variety of microorganisms including *Agrobacterium tumefaciens*. Runser, et al. (Appl. Microb. Biotech. 33:382-388, 1990 and Biotechnology Letters 12[4]:259-264, 1990) describe the isolation of a hydantoinase from an *Agrobacterium* species IP-I671. Although most hydantoinases share the common enzymatic ability to produce N-carbamyl amino acids from 5-mono-substituted hydantoins, there is variability in other characteristics of the enzyme. Lacking in the art is the description of a thermostable hydantoinase isolated from *Agrobacterium* wherein the hydantoinase has a higher relative activity for D,L-5-methylhydantoin or dihydrouracil than for 5(2-methylthioethyl) hydantoin and D,L-5-benzylhydantoin and is not activated by nickel, magnesium, or cobalt. Also lacking in the art is a bacterial strain suitable for producing a novel hydantoinase that exhibits good alkaline and thermal stability properties. Such conditions are favorable for the rapid conversion of DL-hydantoins to the respective N-carbamyl D-amino acids.

### Summary of the Invention

In one embodiment, the present invention is an *A*. *tumefaciens* strain that produces a novel hydantoinase

In another embodiment, the present invention is a preparation of hydantoinase isolated from *A. tumefaciens* with a temperature optimum of greater than 60°C. Preferably, the temperature optimum is greater than 65°C.

Preferably, the hydantoinase of the present invention has a higher relative activity for D,L-5-methylhydantoin and dihydrouracil than for D,L-5-phenylhydantoin and a higher relative affinity for D,L-5-phenylhydantoin than for D,L-5-benzylhydantoin. Preferably, the hydantoinase is not substantially activated by nickel, magnesium, cobalt or other metal ions and is active at high temperatures (>60°C) under alkaline conditions.

The present invention also provides a process for the enzymatic conversion of DL-hydantoins to N-carbamyl D-amino acids and to D-amino acids.

One object of the present invention is to provide a novel and improved hydantoinase that exhibits alkaline and thermal stability properties that are advantageous when the enzyme is used for the production N-carbamyl D-amino acids at high temperatures and in an alkaline medium.

It is another object of the present invention to provide a bacterial strain capable of producing the improved hydantoinase.

It is another object of the invention to provide a method for producing N-carbamyl D-amino acid and D-amino acids from racemic hydantoins.

It is a feature of the present invention that the hydantoinase is more active with D,L-5-methylhydantoin and 5,6-dihydrouracil than with D,L-5-benzylhydantoin and more active with D,L-5-phenylhydantoin than with D,L-5-benzylhydantoin, and that the hydantoinase is not activated by nickel, magnesium, cobalt or other metal ions.

Other objects, features and advantages of the present invention will become apparent after examination of the specification, drawings, and claims.

### Description of the Drawing

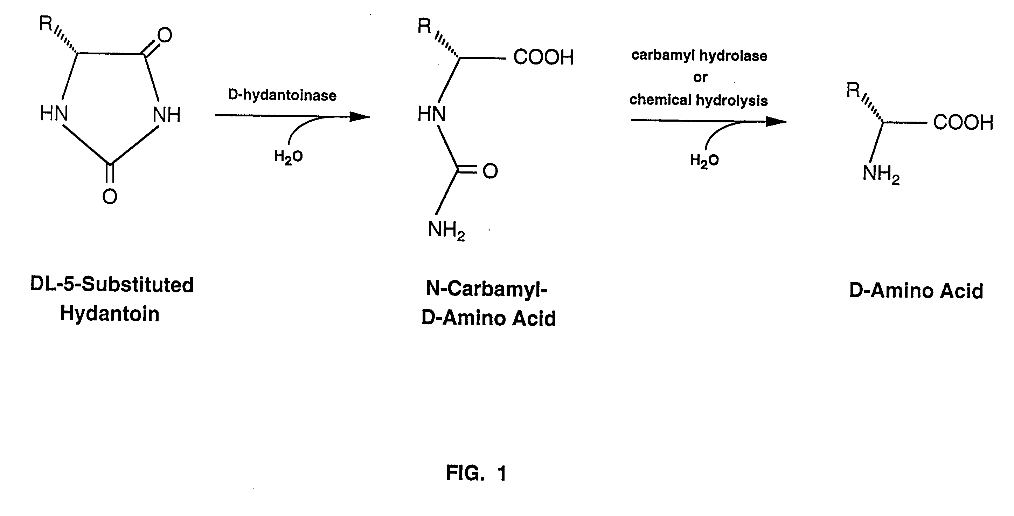

Fig. 1 is a flow chart diagram of the formation of D-amino acid from D,L-5-substituted hydantoin.

### Detailed Description Of The Invention

The present invention is a novel hydantoinase prepared from an environmental bacterial isolate. The *Agrobacterium* species that produces the hydantoinase was isolated from environmental sources using a specific isolation procedure that selected for microorganisms that utilize hydantoins as sole sources of carbon and nitrogen. This isolate was designated 47C. A culture of *A*. *tumefaciens* 47C was deposited with the American Type Culture Collection, Rockville, MD, under the terms and conditions of the Budapest Treaty on November 28, 1994 and has been given accession number ATCC No. 55632.

One embodiment of the present invention is a pure culture of *Agrobacterium tumefaciens* ATCC 55632.

The hydantoinase of the present invention is useful for effecting the conversion of DL-5-monosubstituted hydantoins stereospecifically to N-carbamyl D-amino acids. This conversion is depicted in Fig. 1. The N-carbamyl-D-amino acids thus formed can be further processed to D-amino acids by chemical or enzymatic methods. As will be illustrated below, the hydantoinase of the present invention is characterized by its stability under alkaline conditions, its high activity at temperatures and pH values that favor racemization of the racemic hydantoin substrate, its unique substrate specificity and its independence of metal ions for activity or stimulation of activity.

The hydantoinase is produced by cultivating *A*. *tumefaciens* ATCC 55632 under suitable conditions for expression of the enzyme. The microorganism is typically cultivated under aerobic conditions in a complex medium containing an assimilable source of nitrogen, carbon and trace elements. A variety of growth media can be used for cultivating the strain. An aqueous fermentation medium typically contains 0.1 to 1.0% yeast extract, 0.1 to 1.0% peptone, 0.1 to 1.0% inorganic phosphorous, 0.1 to 1.0% ammonium sulfate, and an assimilable source of carbon such as succinate, glucose or maltose. In addition, various metals salts, such as calcium and magnesium, as well as trace elements are preferably added. The medium typically contains a source of inducer for the hydantoinase, such as hydantoin, 5-methylhydantoin or hydantoic acid at concentrations of 0.1 to 1.0%.

The cultivation medium is vigorously aerated during fermentation and the pH of the medium is maintained between 6.0 to 8.0, preferably at 7.2. The temperature of the fermentation is preferably between 25°C to 37°C, most preferably 28°C. A productive fermentation typically is about 12 to 30 hours in length and most preferably is within the range of 18 to 24 hours.

The hydantoinase of the present invention has a temperature optimum of >60°C, preferably >65°C. Example 3(A) below demonstrates a preferred method of determining a temperature optimum for any candidate hydantoinase.

The hydantoinase of the present invention has a unique substrate specificity described in Example 3(E) below. In particular, the hydantoinase of the present invention has a high relative activity with dihydrouracil and 5-methylhydantoin and a relatively low rate of activity with benzylhydantoin.

The hydantoinase of the present invention is not substantially activated by nickel and is preferably not substantially activated by magnesium, nickel, cobalt or any other metal ions. Example 3(D) below demonstrates a method of assaying metal activation.

The hydantoinase of the present invention is stable in alkaline conditions. Preferably, at least 90% residual activity remains when the hydantoinase is incubated for 24 hours at 25°C at pH 9.0 - 10.0. The hydantoinase has a pH optimum of between pH 8.0 - pH 10.5. Examples 3(B) and 3(C) demonstrate suitable procedures for testing any candidate enzyme.

The hydantoinase of the present invention is induced by 5-methylhydantoin, hydantoin and hydantoic acid. Example 3(F) demonstrates suitable methods to test any candidate enzymes for induction.

The hydantoinase is preferably recovered from the cells after growth by disrupting or lysing the cells and collecting the hydantoinase in the supernatant fraction ("hydantoinase extract") following high speed centrifugation. In one typical preparation procedure, the fermentation broth is first passed through a microfiltration membrane cartridge to concentrate the cells. The cells are further concentrated by centrifugation. The cell pellets are then resuspended in a suitable buffer, preferably Tris-HCl (pH 8.0) and disrupted by various physical methods that include sonication, pressure changes, shear forces, osmotic disruption, and the like. The disrupted cells are further clarified by centrifugation to remove cell debris and unbroken cells. The supernatant fraction containing the hydantoinase extract can be then used for biochemical conversions in accordance with the present invention.

The hydantoinase extract can then be added to aqueous reaction mixtures containing a suitable hydantoin. The reactions are generally performed in vessels that are controlled for both pH and temperature. The pH can range from 8.0 to 11.0, preferably pH 9.0 to 9.5. The temperature of the reaction mixtures can range from 30°C to 75°C, preferably 40°C to 45°C. The pH and temperature are important aspects of the reaction because at higher pH and temperatures the DL-hydantoins are more soluble and are racemized at a faster rate to utilizable substrate, D-isomer, for the hydantoinase. Temperature or pH extremes should be avoided, however due to spontaneous hydrolysis of the hydantoin resulting in undesirable side products.

The hydantoin substrates can be added to concentrations of 0.1% to 30%, depending on the solubility of the substrate. High concentrations of hydantoins can be suspended in solvents such as methanol to 10% concentration to increase their solubility.

Hydantoinase is generally added to a concentration of 0.5 to 10 units per ml, preferably 1.5 units per ml. The formation of the reaction product can be monitored by high performance liquid chromatography (HPLC) or by simple colorimetric assays, as described below and in the Examples. The reactions are generally run until the substrate is converted in its entirety to the N-carbamyl D-amino acid, which may take between 6 to 20 hours depending on the substrate and concentration of substrate.

The N-carbamyl D-amino acid product can be separated from the hydantoinase and other proteins in the reaction mixture by passing the reaction mixture through an Amicon (Beverly, MA) S10Y3 spiral ultrafiltration cartridge or similar product. The deproteinated permeate containing the N-carbamyl D-amino acids can be cleaved to the respective D-amino acid by conventional chemical technology, such as the HONO method of Bayer (Takehahashi, et al., *J*. *Fermentation Technology* 57:328, 1979). The retentate containing the hydantoinase can be used for a subsequent bioconversion (see Example 9).

The microbial bioconversions described in the present invention can be performed with intact whole cells or with extracts prepared from disrupted or lysed cells. Furthermore, the cells can be immobilized by conventional procedures know to one of skill in the art (see, for example, Immobilized Enzymes For Industrial Reactors, 1975, Ed. R. Messing Academic Press, NY. and Immobilized Microbial Cells ACS Symposium Series 106, 1979, K. Venkatsubramanian, American Chemical Society, Washington, DC.). Immobilization may involve the covalent attachment or entrapment of cells or hydantoinase extracts or may involve the adsorption of cells to inorganic or organic surfaces.

The hydantoinase extract or appropriately induced whole cells can be immobilized by covalent attachment onto various support matrices such as agarose, cellulose, dextran, glass, HYPOL® (Hamphshire Chemicals, Lexington, MA), polyacrylamide co-polymers or polystyrene or by entrapment with alginate, carrageenan, agar, polyacrylamide or microencapsulated within polymer membranes such as cellulose nitrate/acetate, nylon, lipid polyamide and the like or by adsorption to inorganic or organic surfaces such as alumina, kaolin, ion-exchange resins, porous glass, clay, cellulose, collagen, calcium phosphate gel, bentonite, carbon or Type Z and Type CZ biocarriers (W. R. Grace & Co.). After the reaction is complete the immobilized enzyme can be physically separated from the reaction mixture and used for subsequent reactions (see Example 11).

The invention disclosed and claimed herein is further illustrated by the following examples which are presented for illustrative purposes only and not to be construed as limiting.

### Example 1

**Fermentation of *A*. *tumefaciens***. Fermentations of *A*. *tumefaciens* ATCC 55632 were conducted in 15-liter Biolafitte fermenters with a 12-liter working volume of medium of the following composition (in grams or milliliters per liter): Bacto-peptone (Difco), 10; Bacto-yeast extract, 10; K₂HPO₄, 5; (NH₄)₂SO₄, 5; trace elements, 7.1 ml (from U.S. patent 5,145,681), and polyglycol P-2000, 0.4 ml. After sterilization, 20 mM sodium succinate and 1 g/l (final concentration) of DL-hydantoin were added to the medium (28°C) from sterile (filter sterilized), concentrated stock solutions. Each fermentor was inoculated with a 1% seed culture of *Agrobacterium* grown in 700 ml of same medium in a 2-liter Fernbach flask. Fermentations were performed at 28°C, pH 7.2, 1,000 rpm, and aerated at 1.0 volume of air per volume of medium per minute. The pH was maintained with acid and base titrants consisting of 10% phosphoric acid and 2 N NaOH, respectively. Cell growth was followed spectrophotometrically at 600 nm.

### Example 2

**Preparation of hydantoinase**. Approximately 60 liters of *A*. *tumefaciens* cells were collected from 5 fermentors using microfiltration (AG Technology Corp., Needham, MA/0.45 µm exclusion limit cartridge) and concentrated to a 2.5 liter cell slurry. The cell slurry was further harvested by centrifugation at 10,000 x g at 4°C, and the pellets were stored at -80°C until use.

Cell pellets were resuspended in 2 volumes of 50 mM Tris-HCl buffer, pH 8.0 and disrupted at 20,000 psi by passage through a French pressure cell (Amico Corp.) operated in a continuous mode. Cell debris was removed by centrifugation at 37,500 x g for 1 hour at 4°C. The supernatant fraction (hydantoinase extract) was used immediately or stored at -80°C until use.

### Example 3

### Properties of Hydantoinase From A. Tumefaciens ATCC 55632

**A. Temperature/activity relationship.** Hydantoinase activity was measured using a modification of the colorimetric procedure for detecting N-carbamyl amino acids with *p*-dimethylaminobenzaldehyde (PDMB) as described by Cecere, et al. (FEBS Letters, 57:192 (1975)). Reaction mixtures (total vol. of 0.4 ml in microfuge tubes) contained 75 µmol of DL-5-methylhydantoin (unless designated otherwise) in 50 mM Tris-HCl buffer (pH 9.0) and a suitable amount of hydantoinase extract. Hydantoinase extracts were added to reaction mixtures which had been pre-equilibrated at 40°C, and after 10 minutes incubation at this temperature, reactions were terminated by the addition of 100 µl of 10% (weight/volume) trichloroacetic acid, followed by 100 µl of 10% (weight/volume) PDMB in 6 N HCl. The solutions were mixed, diluted with 0.6 ml of water and centrifuged at 10,000 rpm for 5 minutes. The absorbencies of the supernatant fractions were determined at 440 nm with a Shimadzu Model 160 spectrophotometer. One unit of activity is defined as the amount of enzyme required to form 1 µmol of N-carbamyl amino acid under these conditions. Protein was determined by the BCA assay as per the manufacturer's instructions. (Pierce Chemical Co., Rockford, IL). Specific activity is expressed as units per mg protein.
For the determination of the effect of temperature on hydantoinase activity, hydantoinase extracts of *A*. *tumefaciens* were added to reaction mixtures pre-equilibrated at various temperatures and activities were determined by the aforementioned assay. Controls (minus enzyme) were run for each temperature and the values obtained were subtracted from the test sample. Results are tabulated in Table 1.

**TABLE 1**

| **Temperature (°C)** | **Specific Activity** | **Relative Activity (%)** |
|---|---|---|
| 30 | 0.49 | 28 |
| 40 | 0.81 | 46 |
| 50 | 1.22 | 70 |
| 60 | 1.50 | 86 |
| 65 | 1.65 | 94 |
| 70 | 1.75 | 100 |
| 75 | 1.49 | 85 |

**B. pH/activity relationship.** For determination of hydantoinase activity as a function of pH, hydantoinase activity of *A. tumefaciens* ATCC 55632 was determined in reaction buffers over a pH range of 6.0 to 11.0 at 40°C following the standard hydantoinase assay as previously described with controls (no enzyme) for each pH value. The buffers (50 mM) used were phosphate (pH 6.0, 7.0, 8.0), Tris-HCl (pH 9.0), and [3-(cyclohexylamino) propane sulfonic acid] (CAPS) (pH 10.0, 10.5, 11.0). Results are tabulated in Table 2.

**TABLE 2**

| **pH** | **Specific activity** | **Relative activity** |
|---|---|---|
| 6 | 0.17 | 17 |
| 7 | 0.50 | 50 |
| 8 | 0.76 | 76 |
| 9 | 0.87 | 87 |
| 10 | 1.04 | 100 |
| 10.5 | 0.80 | 80 |
| 11 | 0.62 | 60 |

**C. pH/stability relationship.** To determine the pH stability of hydantoinase, hydantoinase extracts were incubated at 25°C for 24 hours in buffers formulated at various pH values. Hydantoinase extract was added to the stock buffer solutions and were assayed immediately to give an initial activity (time zero). After the incubation period (24 hours), the enzyme solutions were assayed again to determine the residual hydantoinase activity. For all assays, the enzyme solutions were allowed to equilibrate at 37°C, and reactions were initiated by the addition of a concentrated stock of DL-5-methylhydantoin to give a final substrate concentration of 10 mg/ml. The buffers used were 50 mM MES (2[N-morpholino]ethane sulfonic acid) (pH 6.0), 50 mM Tris-HCl (pH 7.0, 7.5, 8.0, 8.5), 25 mM Borate (pH 9.0) and 50 mM CAPS (pH 10.0, 10.5).
The pH stability profile is tabulated in Table 3.

**TABLE 3**

| **pH** | **Residual Act. (%)** |
|---|---|
| 6 | 58 |
| 7 | 74 |
| 7.5 | 83 |
| 8 | 90 |
| 8.5 | 88 |
| 9 | 98 |
| 10 | 90 |
| 10.5 | 76 |

**D. Hydantoinase activation by metals.**
Hydantoinase extract of *A. tumefaciens* ATCC 55632 was dialyzed against 50 mM Tris-HCl buffer. Solutions (360 µl) containing either 2 mM Mg⁺⁺, Co⁺⁺ or Ni⁺⁺ in 50 mM Tris buffer, pH 9.0 were pre-incubated with dialyzed hydantoinase extract for 30 minutes at ambient temperature. The reaction mixtures were equilibrated in a 40°C water bath for 2 minutes. The hydantoinase assay was started immediately with the addition of 40 µl DL-5-methylhydantoin (100 mg/ml in 50 mM Tris-HCl buffer, pH 9.0). The results in Table 4 below show the effect of these metal ions on hydantoinase activity.

**TABLE 4**

| **Addition** | **Hydantoinase Activity (Units/ml)** |
|---|---|
| None (Control) | 26.4 |
| Magnesium | 24.8 |
| Nickel | 25.0 |
| Cobalt | 27.9 |

For comparative purposes, the hydantoinase of the present invention was compared in Table 5, below, to other hydantoinases from microbial sources that are described in the literature; 1). *Arthrobacter crystallopoietes* AM2 [Enzyme Microb. Technol. 10:618 (1988)], 2). *Pseudomonas fluorescens* DSM 84 [Biotechnol. Appl. Biochem. 8:564 (1986], 3). *P. striata* [J. Fermentation Technol. 56:492 (1978)], 4). *Pseudomonas* sp. AJ 11220 [Agric. Biol. Chem. 51:721 (1987], 5). *Agrobacterium radiobacter* [Biotechol. Bioengineering 23:2173 (1981)], and 6). *Agrobacterium* IP I671 [Biotechnol. Lett. 12:259 (1990)].

**TABLE 5**

| **Property** | **Source of hydantoinase** | | | | | | | |
|---|---|---|---|---|---|---|---|---|
| | **1** | **2** | **3** | **4** | **5** | **6** | **ATCC 55632** | |
| Temp. optima(°C) pH | 50-60 | 55 | 45-55 | 55 | 60 | 55-60 | 70 | |
| optima | 8.2-9.2 | 9.0 | 8-9 | 8 | 9 | 10 | 10 | |
| Metal ion requirement | none | Fe²⁺ | Fe²⁺ | -- | none | Ni²⁺ | none | |
| | | Co²⁺ | | | | Mg²⁺ | | |
| pH stability | 6.5 | 5.5-8.5 | 6-7 | -- | < 8 | 8-10 | 7-10 | |

Referring to Table 5, the hydantoinase of the present invention differs from previously described hydantoinases in its combination of high temperature and pH optima, pH stability and lack of requirement or stimulation by metal ions. Additionally, no other tested hydantoinase in Table 5 had a temperature optimum >60°C.
**E. Substrate specificity.**
The substrate specificity of the *A. tumefaciens* ATCC 55632 hydantoinase was determined by adding hydantoinase extract to 0.4 ml of substrate solution (5 mg/ml 50 mM Tris-HCl buffer, pH 9.0) and incubating the reaction mixtures at 40°C in a water bath for 10 minutes in sealed tubes. The reactions were terminated and further processed as described previously. Controls (minus enzyme) were run for each substrate the values obtained were subtracted from the test sample. The results are shown in Table 6.

**TABLE 6**

| **Substrate** | **Relative Activity (%)** |
|---|---|
| DL-5-Methylhydantoin | 100 |
| 5,6-Dihydrouracil | 125 |
| DL-5-Phenylhydantoin | 85 |
| 5(2-methylthioethyl) hydantoin | 81 |
| DL-5-Isobutylhydantoin | 77 |
| DL-5-(sec)-Butylhydantoin | 76 |
| DL-5-Isopropylhydantoin | 73 |
| DL-5-Hydroxymethylhydantoin | 22 |
| DL-5-Benzylhydantoin | 19 |

By comparison, the substrate specificity of the hydantoinase of the present invention was compared in Table 7 below to hydantoinases reported in the literature: 1) *Arthrobacter crystallopoietes* AM2 [Enzyme Microb. Technol. 10:618 (1988)], 2). *Pseudomonas fluorescens* DSM 84 [Biotechnol. Appl. Biochem. 8:564 (1986)], 3). *P. striata* [J. Fermentation Technol. 56:492 (1978)], 4). *Pseudomonas* sp. AJ 11220 [Agric. Biol. Chem. 51:721 (1987)], 5). *Agrobacterium radiobacter* [Biotechol. Bioengineering 23:2173 (1981)], and 6). *Agrobacterium* IP I671 [Biotechnol. Lett. 12:259 (1990)].

**TABLE 7**

| **Hydantoin** | % Relative Activity with: | | | | | | |
|---|---|---|---|---|---|---|---|
| | **1** | **2** | **3** | **4** | **5** | **6** | **ATCC 55632** |
| DL-5-Methyl | 100 | nd | 45 | nd | 24 | 37 | 80 |
| 5,6-Dihydrouracil | 78 | 100 | 100 | 9 | nd | 10 | 100 |
| DL-5-Phenyl | 37 | nd | 25 | 34 | 51 | nd | 68 |
| 5(2-methylthioethyl) | nd | 41 | 48 | 100 | 100 | 55 | 65 |
| DL-5-Benzyl | nd | 2 | nd | 57 | 32 | 100 | 15 |
| nd= not disclosed. | | | | | | | |

These comparisons demonstrate that the hydantoinase from *A. tumefaciens* ATCC 55632 has novel substrate specificities compared with those previously described in the literature. In particular it should be noted that the substrate specificity of hydantoinase from *A. tumefaciens* ATCC 55632 differs substantially from those described for other *Agrobacterium* species (5 and 6) due to its high relative activity with dihydrouracil and 5-methylhydantoin and its relatively low rate of hydrolysis with 5-benzylhydantoin
**F. Induction of hydantoinase activity.**
*A. tumefaciens* ATCC 55632 was grown in a medium containing basal salts medium, 1% yeast extract and 20 mM glucose. Various hydantoins or carbamyl amino acids were added to a concentration of 2 mg/ml to induce hydantoinase activity. Cells were harvested after 8 hours, hydantoinase extracts were prepared and hydantoinase activity was determined. Table 8 demonstrates the effect of these chemicals on enzyme induction.

**TABLE 8**

| **Addition** | **Specific Activity (U/mg)** |
|---|---|
| None | 0.06 |
| 1-Methylhydantoin | 0.09 |
| 5-Methylhydantoin | 1.65 |
| Hydantoin | 1.44 |
| Tryptophan hydantoin | 0.10 |
| Dihydrouracil | 0.22 |
| Hydantoic acid | 1.34 |
| N-carbamyl alanine | 0.44 |
| N-carbamylphenylalanine | 0.13 |

2,4-Thiouracil (2,4-dithiouracil) has been reported to induce hydantoinase activity in Agrobacterium IP I-671 by 40-fold [Meyer, et al., FEMS 109:71(1993)]. In a separate experiment, hydantoinase activities were determined following the growth of *A. tumefaciens* ATCC 55632 on a complex medium (750 ml) (see media preparation described in Example 1) containing the inducing compounds at 1 gm/liter. The culture media were inoculated with 24 hour seed culture of *A. tumefaciens* ATCC 55632 and incubated for 24 hours with shaking at 30°C. Hydantoinase extracts were prepared and the effect of 2,4-thiouracil on hydantoinase induction of *A. tumefaciens* is shown in Table 9.

**TABLE 9**

| **Inducer** | **Specific Activity (Units/mg)** |
|---|---|
| None | 0.13 |
| 2,4-Thiouracil | 0.15 |
| Hydantoin | 1.37 |

These data suggest that the induction of *A. tumefaciens* ATCC 55632 differs significantly from that of *Agrobacterium* IP I-671 in that 2,4-thiouracil does not appear to induce hydantoinase activity in *A. tumefaciens* ATCC 55632. The addition of hydantoin, however, increased hydantoinase activity 10-fold. Furthermore, this compound does not induce hydantoinase activity in *Agrobacterium* IP I-671. (FEMS 109:71, 1993)

### Example 4

**Taxonomy of Agrobacterium Tumefaciens ATCC 55632.** For taxonomic purposes, use was made of Bergey's Manual of Systematic Bacteriology vol. 1 (1984) and several recent publications concerning the taxonomic status of the genus *Agrobacterium* (Holmes and Rhodes, J. Appl. Bacteriol. 50:443-467, 1981; Bouzsar, et al., Appl. Environ. Microbiol. 61:65-73, 1995 and Phytopathology 83:733-739, 1993; Bouzar, Int. J. Syst. Bacteriol. 43:694-702, 1993).

*A. tumefaciens* ATCC 55632 was isolated by enrichment culture using DL-5-methylhydantoin as a sole carbon and nitrogen source. The characteristics of the isolate are consistent with its identification with biovar 1 of *Agrobacterium* or *Agrobacterium tumefaciens* as determined by substrate utilization pattern (Biolog), fatty acid composition and biochemical properties. The strain is oxidase positive, produces 3-keto-lactose on lactose medium, does not utilize citrate, grows on MacConkey's agar and at temperatures to 37°C. The isolate was identified as *A. tumefaciens* using the Vitec system (Vitex Systems Industries, Hazelwood, MI) and by fatty acid analysis (Acculabs, Newark, DE) and *A. rhizogenes* with the Biolog system (Hayward, CA). The Biolog substrate utilization pattern corresponds to those reported for *A. tumefaciens* (Bouzar, et al. 1993. Phytopathology 83:733-739) with the exception that strain ATCC 55632 oxidizes D-glucosaminic acid and alaninamide. Furthermore, the strain differs from 80% of the *A. tumefaciens* strains analyzed by Bouzar, et al., Appl. Environ. Microbiol. 61:65-73, 1995, due to its ability to oxidize α-ketoglutaric acid, inosine, and uroncanic acid.

The differences between *A. tumefaciens* ATCC 55632 and other *Agrobacterium* strains previously shown to produce D-hydantoinases are shown in Table 10.

**TABLE 10**

| **Property** | ***Agrobacterium*** **Strain** | | |
|---|---|---|---|
| | **ATCC 55632**^{**a**} | **N1302**^{**a**} | **IP-I671**^{**b**} |
| ONPG hydrolysis^{c} | - | + | + |
| 3-ketolactose | + | + | - |
| Hydroxy-butyric acid | + | - | ND |
| Citric acid | - | - | + |

| | | | |
|---|---|---|---|
| a Our determination | | | |
| b European Patent Application 0 309 310 (1988) | | | |
| c O-nitrophenyl-β-D-galactopyranoside | | | |

### Other Taxonomic Properties:

Microscopic cell morphology: Gram negative, motile rods. 0.5 to 0.8 µm wide by 1 to 3 µm in length. Non-sporeformer.

Colony morphology: Colonies on TSA agar are beige/cream colored, circular, convex, smooth colonies with entire edges.

### Biochemical Characteristics:

Strain grows on basal salts medium without additional growth factor or vitamins and can use hydantoins and amino acids as sole carbon and nitrogen sources. The strain grows to temperatures to 37°C.

The following characteristics were determined using the Vitec system for microbial identification:

Aerobic acid production from: glucose, lactose, maltose, mannitol and xylose.

Fermentative production of acid from: adonitol (+), 1-arabinase (+), raffinose (-), sorbitol (-), sucrose (-), inositol (-), rhamnose (-), glucose (-).

Other Biochemical properties: citrate (-), acetamide (-), oxidase (+), catalase (+), malonate (-), indole (-), H₂S (-), esculin (+), urease (+), β-galactosidase (-), lysine and ornithine decarboxylase (-), arginine dihydrolase (-).

The fatty acid composition of *A. tumefaciens* ATCC 55632 is consistent with that described for Cluster A of *A. tumefaciens* as reported by Bouzar and co-workers (Phytopathology 83:733-739, 1993). The fatty acid composition of the isolate ATCC 55632 is as follows: 14:0 (0.2%), 16:1_{*ω7cis*} (3.9%), 16:0 (8.1%), 17:0cyc (0.4%), 3-OH-16:0 (3.3%), 18:0 (0.2%), 19:0cyc_{*ω8cis*} (4.4%), 19:0-10-methyl (0.7%), 3-OH-14:0 (sum of Acculab feature 3; 5.8%), and 18:1_{*ω7cis*} (sum of Acculab feature 7; 73.4%).

### Example 5

**Formation of N-carbamyl D-alanine.** The conversion of DL-5-methylhydantoin to N-carbamyl D-alanine was achieved using a Metorholm pH stat/autotitration system (Brinkmann Instruments). Reactions were performed in 50 ml jacketed vessels with temperature control and pH controlled with 0.5 N NaOH. Hydantoin substrate was added to reaction mixtures (10 ml) to a concentration of 20 g/l. The reaction was maintained at pH 9.0, and equilibrated at 40°C before extract addition. Hydantoinase extract (1.6 Units/ml) was added, and the reactor was sampled for N-carbamyl amino acid formation colorimetrically with PDMB.

Table 11 demonstrates this conversion.

**TABLE 11**

| **Hour** | **g/l Product** | **% Conversion** |
|---|---|---|
| 0 | 0 | 0 |
| 0.5 | 6.6 | 28 |
| 1 | 11.4 | 49 |
| 2 | 16.2 | 69 |
| 3 | 17.1 | 74 |
| 4 | 20.5 | 89 |
| 6 | 24.7 | 100 |

The specific rotation of the product (5 mg/ml) was -12.8 as determined with a Perkin Elmer polarimeter model #241 with a sodium lamp (589).

### Example 6

**Formation of N-carbamyl D-phenylglycine.** The conversion of DL-phenylhydantoin to N-carbamyl D-phenylglycine was performed under the reaction conditions described in Example 5, except for the change in substrate. Table 12 summarizes this conversion.

**TABLE 12**

| **Minutes** | **g/l product** | **% Conversion** |
|---|---|---|
| 0 | 0 | 0 |
| 10 | 8.0 | 37 |
| 20 | 12.6 | 58 |
| 45 | 13.3 | 62 |
| 60 | 14.8 | 68 |
| 90 | 17.7 | 82 |
| 180 | 19.2 | 89 |
| 300 | 20.63 | 96 |

The optical rotation of the N-carbamyl phenylglycine reaction product was determined using a Perkin Elmer polarimeter Model # 241 with a sodium lamp (589). Four mg/ml of DL-5-phenylhydantoin (Toronto Research Chemicals Inc., Canada) in 0.1 M Na₂HPO₄, pH 9.5, was combined with 100 µl of filtered sterilized hydantoinase extract. The optical and specific rotation was followed as a function of time. The results are shown in Table 13.

**TABLE 13**

| **Minutes** | **Observed Rotation** | **Specific Rotation** |
|---|---|---|
| 0 | 0 | 0 |
| 5 | -0.034 | -8.5 |
| 10 | -0.084 | -21.0 |
| 20 | -0.179 | -44.8 |
| 30 | -0.247 | -61.8 |
| 40 | -0.293 | -73.3 |
| 68 | -0.365 | -91.3 |
| 86 | -0.386 | -96.5 |
| 120 | -0.408 | -102.0 |

The final specific rotation of recovered product was -127.6, which compares favorably with the published rotary power for D-(-)-N-carbamyl phenylglycine ([α]_{D}²⁰ = - 136.3) described in Chemical Abstracts, 65:383d (1966).

### Example 7

**Formation of diethylphosphonate (DEP) carbamyl D-amino acid.** DL-DEP hydantoin was used as a starting substrate for the conversion to DEP carbamyl amino acid. The reaction was performed in a temperature controlled vessel at 40°C with mixing. The pH of the solution was maintained at 9.5 with a 1 N NaOH solution using a pH controller. DL-DEP hydantoin in methanol was added to the reaction mixture (15 liter) to a concentration of 1.46% by weight hydantoin resulting in a final reactor volume of 17.44 liters. After equilibration and final pH adjustment, the hydantoinase extract was added to a concentration of 1.6 units per ml. The formation of DEP carbamyl amino acid was followed by HPLC. Analytical HPLC analysis was performed with a Perkin-Elmer HPLC. Separations were accomplished with a Supelco LC-18 column (150 mm by 4.6 mm) at a flow rate of 1 ml/min in an isocratic system with the mobile phase containing 25% acetonitrile, 3 mM n-heptanesulfonic acid, 3.8 mM phosphoric acid, 5 mM sec-butylamine and 1.6 mM ammonium acetate. Detection was at 195 nm with a Perkin-Elmer 235C-diode array detector. A summary of the conversion is shown in Table 14.

**TABLE 14**

| **Hour** | **DEP carbamyl D-amino acid (g/l)** |
|---|---|
| 0 | 0 |
| 1 | 4.7 |
| 2 | 7.6 |
| 3 | 8.84 |
| 5 | 10.4 |
| 7 | 10.95 |
| 9 | 11.78 |
| 23.5 | 13.18 |

### Example 8

**Isolation and crystallization of DEP-carbamyl-D-amino acid.** The N-carbamyl amino acid reaction product was isolated by preparative HPLC for determination of the absolute stereo-chemistry. A product solution was applied to a Waters Delta Prep 3000 HPLC. Separations were accomplished with a Supelco PLC-18 column (25 cm by 2.1 cm/12 micron packing) at a flow rate of 1 ml/min in an isocratic system. The mobile phase contained 27% acetonitrile, 0.3% acetic acid, and 0.025% ammonium acetate. Detection was at 225 nm with a Waters UV detector. Fractions of 5 ml were collected, and those fractions containing carbamyl amino acid were pooled and rechromatographed. The rechromatographed fractions were pooled (76 ml) and concentration by rotoevaporation at 40°C to a 5 ml volume. The solution was cooled at room temperature forming crystals. The suspension was filtered and 52.8 mg of white, needle-shaped crystals of DEP carbamyl D-amino acid were recovered. A single crystal was used to demonstrate the absolute stereochemistry as 2R, 4S, 5S [J. Org. Chem. 58:7263-7370 (1993)].

### Example 9

**Hydantoinase recovery and reuse.** At the conclusion of a 17 liter DL-DEP hydantoin enzymatic conversion, the reactor solution was passed through a spiral wound membrane (S10Y3, Amicon Corp.) cartridge to remove protein, including hydantoinase. The hydantoinase, recovered in the retentate (1,990 ml) was further concentrated by passage through a S1Y10 spiral wound membrane (Amicon Corp., Beverly, MA). The hydantoinase extract was diafiltered against 2-liters of deionized water and concentrated to a final volume of 516 ml. The recovery of the hydantoinase activity is shown in Table 15, below.

**TABLE 15**

| **Fraction** | **u/ml** | **Total Vol.** | **Total U** | **% Recovery** |
|---|---|---|---|---|
| S10Y3 Retentate | 24.1 | 1,990 | 47,959 | 100 |
| S1Y10 Retentate | 90.2 | 516 | 46,543 | 97 |

An aliquot of the recycled hydantoinase preparation was used for the conversion of DL-DEP hydantoin and compared to freshly prepared hydantoinase extract (control) at the same enzyme concentration. The results shown below in Table 16 indicate that *A. tumefaciens* hydantoinase can be successfully recovered and used for additional conversions.

**TABLE 16**

| **Hour** | **DEP carbamyl D-amino acid formed with:** | |
|---|---|---|
| | **Recycled Hydantoinase** | **Control Hydantoinase** |
| 0 | 0 | 0 |
| 1 | 2.0 | 3.1 |
| 2 | 5.2 | 6.3 |
| 5 | 7.7 | 8.7 |
| 7 | 9.0 | 9.5 |
| 23 | 11.1 | 12.3 |

### Example 10

**DL-DEP hydantoin conversion using whole cells of** ***A. tumefaciens.*** Reactor conditions were established as described in Example 5. DL-DEP hydantoin concentration was 1.5%. Whole cells (0.8 g) of *Agrobacterium* (390 units of activity per g of cells) were added to the reaction mixture. Reaction product was followed as per Example 7. Table 17 demonstrates the conversion with whole cells.

**TABLE 17**

| **Hour** | **g/l product** | **% Conversion** |
|---|---|---|
| 0 | 0 | 0 |
| 0.5 | 2.48 | 9.2 |
| 1.5 | 4.88 | 15.7 |
| 2.0 | 6.5 | 30.9 |
| 4.5 | 9.6 | 60.8 |
| 5.5 | 10.98 | 69.5 |
| 7.0 | 11.61 | 73.5 |
| 22.0 | 15.21 | 96.3 |

The whole cell reaction had an initial rate of 3.3 g/l per hour product formation and 96.3% conversion at 23 hours.

### Example 11

**Immobilization of whole cells and demonstration of reuse.** *A. tumefaciens* was immobilized within Hypol® foam (Hampshire Chemicals, Lexington, Mass.) and used for the conversion of DL-5-methylhydantoin to N-carbamyl D-alanine. Four grams of cell paste were mixed with 0.5 ml phosphate buffer, pH 8.5. The cell slurry was then mixed with 3.7 g of Hypol® FHP-3000 HD with rapid mixing to allow the slurry to foam and cure. The foam was then cut into small pieces (0.5 cm), washed 2 times in 200 ml of phosphate buffer and pulled dry with vacuum through a Buchner funnel. Reaction mixtures were as described in Example 5 with 20 g/l of DL-5-methylhydantoin as substrate. Immobilized cells, 3.43 g of foam containing 1.41 g of cell paste, were added to the reaction mixture, and the reaction was run for 6 hours during which the substrate was completely converted to the carbamyl amino acid.

After each run, foams were rinsed with phosphate buffer and pulled dry with vacuum as before. Foam samples were stored at 4°C between runs. For subsequent runs, the foams were added to reactions mixtures as before and the carbamyl amino acid product formation was monitored. This process was continued for seven reuse cycles of the immobilized cells. A summary of the immobilized whole cell bioreactor runs demonstrating reuse of the biocatalyst is shown in Table 18.

**TABLE 18**

| **Run #** | **Initial Rate (g/l per hour)** | **% of Initial Rate** | **% Conversion at 3 Hours** |
|---|---|---|---|
| 1 | 15.7 | 100 | 76 |
| 2 | 15.0 | 96 | 88 |
| 3 | 14.9 | 95 | 92 |
| 4 | 14.0 | 89 | 95 |
| 5 | 13.4 | 85 | 90 |
| 6 | 11.7 | 75 | 82 |
| 7 | 10.6 | 68 | 88 |
| 8 | 9.4 | 60 | 78 |

## Claims

1. A preparation of hydantoinase isolated from *Agrobacterium tumefaciens*, said hydantoinase having a temperature optimum of greater than 60°C.

2. The hydantoinase preparation of claim 1, wherein the hydantoinase has the following relative activities for the following substrates: 5,6 dihydrouracil > D,L-5-methylhydantoin, D,L-5-phenylhydantoin > D,L-5-isobutylhydantoin > D,L-5-hydroxymethylhydantoin > D,L-5-benzylhydantoin.

3. The hydantoinase preparation of claim 1 wherein the hydantoinase is not activated by nickel.

4. The hydantoinase preparation of claim 1 wherein the hydantoinase is most active between pH 8.0 to 10.5.

5. The hydantoinase preparation of claim 1 wherein the hydantoinase is derived from *Agrobacterium tumefaciens* ATCC 55632.

6. A hydantoinase isolated from ATCC 55632.

7. The hydantoinase of claim 6, wherein the hydantoinase has the following relative activities for the following substrates: 5,6 dihydrouracil > D,L-5-methylhydantoin, D,L-5-phenylhydantoin > D,L-5-isobutylhydantoin > D,L-5-hydroxymethylhydantoin > D,L-5-benzylhydantoin.

8. The hydantoinase of claim 6 wherein the hydantoinase is not activated by nickel.

9. The hydantoinase of claim 6 wherein the hydantoinase is most active between pH 8.0 to 10.5.

10. A pure culture of *Agrobacterium tumefaciens* ATCC 55632.

11. A method of converting 5-monosubstituted hydantoins to N-carbamyl D-amino acids comprising the step of subjecting the 5-monosubstituted-hydantoins to the hydantoinase preparation of claim 1 under temperature and buffer conditions permitting the conversion of 5-monosubstituted hydantoins to N-carbamyl D-amino acids.

12. The method of claim 11 wherein the conversion is done in an aqueous medium using hydantoinase provided from a crude cell extract.

13. The method of claim 11 wherein the hydantoinase is provided from intact cells.

14. The method of claim 13 further comprising the step of using the intact cells for consecutive conversions.

15. The method of claim 13 wherein the intact cells are immobilized.

16. The method of claim 15 wherein the immobilized cells are used for consecutive conversions of DL-hydantoins to N-carbamyl D-amino acids.

17. The method of claim 12 wherein the cell extract is immobilized.

18. The method of claim 11 wherein the hydantoinase is provided by a microorganism grown in a nutrient medium containing an inducer to induce hydantoinase production.

19. The method of claim 18 wherein the inducer is selected from the group of hydantoin, DL-5-methylhydantoin and hydantoic acid.

20. The method of claim 11 wherein the hydantoinase is provided by *Agrobacterium tumefaciens* ATCC 55632.

21. The method of claim 11 further comprising the step of converting the N-carbamyl D-amino acids to D-amino acids.

22. A method of converting 5-monosubstituted hydantoins to N-carbamyl D-amino acids comprising the step of subjecting the 5-monosubstituted-hydantoins to the hydantoinase preparation of claim 6 under temperature and buffer conditions permitting the conversion of 5-monosubstituted hydantoins to N-carbamyl D-amino acids.

23. The method of claim 22 wherein the conversion is done in an aqueous medium using hydantoinase provided from a crude cell extract.

24. The method of claim 22 wherein the hydantoinase is provided from intact cells.

25. The method of claim 24 further comprising the step of using the intact cells for consecutive conversions.

26. The method of claim 24 wherein the intact cells are immobilized.

27. The method of claim 26 wherein the immobilized cells are used for consecutive conversions of DL-hydantoins to N-carbamyl D-amino acids.

28. The method of claim 23 wherein the cell extract is immobilized.

29. The method of claim 22 wherein the hydantoinase is provided by a microorganism grown in a nutrient medium containing an inducer to induce hydantoinase production.

30. The method of claim 29 wherein the inducer is selected from the group of hydantoin, DL-5-methylhydantoin or hydantoic acid.

31. The method of claim 22 further comprising the step of converting the N-carbamyl D-amino acids to D-amino acids.
